(19) Europäisches Patentamt / European Patent Office / Office européen des brevets

(11) **EP 1 667 537 B1**

(12) **EUROPEAN PATENT SPECIFICATION**

(45) Date of publication and mention
of the grant of the patent:
**06.11.2013 Bulletin 2013/45**

(21) Application number: **04762842.5**

(22) Date of filing: **17.09.2004**

(51) Int Cl.:
*A23L 1/29* (2006.01)   *A23L 1/305* (2006.01)

(86) International application number:
**PCT/DK2004/000622**

(87) International publication number:
**WO 2005/025333 (24.03.2005 Gazette 2005/12)**

(54) **INFANT FORMULA**

FERTIGNAHRUNG FÜR KLEINKINDER

FORMULE POUR NOURRISSON

(84) Designated Contracting States:
**AT BE BG CH CY CZ DE DK EE ES FI FR GB GR HU IE IT LI LU MC NL PL PT RO SE SI SK TR**
Designated Extension States:
**AL LT LV MK**

(30) Priority: **18.09.2003 DK 200301353**
**23.09.2003 US 504952 P**

(43) Date of publication of application:
**14.06.2006 Bulletin 2006/24**

(73) Proprietor: **Arla Foods Amba**
**8260 Viby J (DK)**

(72) Inventors:
- **SOERENSEN, Esben, Skipper**
  **DK-8471 Sabro (DK)**
- **BURLING, Hans**
  **S-22475 Lund (SE)**
- **BOETTCHER, Karen**
  **DK-6933 Kibaek (DK)**
- **BERTELSEN, Hans**
  **DK-6920 Videbaek (DK)**
- **ALBERTSEN, Kristian**
  **6920 Videbaek (DK)**
- **GRAVERHOLT, Gitte**
  **DK-8200 Aerhus N (DK)**
- **JOERGENSEN, Anders, Steen**
  **DK-8000 Aerhus C (DK)**
- **SCHACK, Lotte**
  **DK-8240 Risskov (DK)**

(74) Representative: **Zacco Denmark A/S**
**Hans Bekkevolds Allé 7**
**2900 Hellerup (DK)**

(56) References cited:
**WO-A-01/11990         WO-A-02/28413**
**US-A1- 2003 149 249**

- **DATABASE BIOSIS [Online] BIOSCIENCES INFORMATION SERVICE, PHILADELPHIA, PA, US; June 2003 (2003-06), CHOI K W ET AL: "Purification and properties of osteopontin from bovine milk." XP002281089 Database accession no. PREV200300375294 & JOURNAL OF ANIMAL SCIENCE AND TECHNOLOGY, vol. 45, no. 3, June 2003 (2003-06), pages 491-498,**
- **DHANIREDDY R ET AL: "Osteopontin in human milk from mothers of premature infants" ACTA PAEDIATRICA, vol. 82, no. 10, 1993, pages 821-822, XP008030887 ISSN: 0803-5253 cited in the application**
- **BAYLESS K J ET AL: "Isolation and Biological Properties of Osteopontin from Bovine Milk" PROTEIN EXPRESSION AND PURIFICATION, ACADEMIC PRESS, SAN DIEGO, CA, US, vol. 9, no. 3, April 1997 (1997-04), pages 309-314, XP004466987 ISSN: 1046-5928**
- **SORENSEN S ET AL: "Purification and characterization of osteopontin from human milk" PROTEIN EXPRESSION AND PURIFICATION, ACADEMIC PRESS, SAN DIEGO, CA, US, vol. 30, no. 2, August 2003 (2003-08), pages 238-245, XP004439533 ISSN: 1046-5928 cited in the application**
- **PATENT ABSTRACTS OF JAPAN vol. 1997, no. 11, 28 November 1997 (1997-11-28) & JP 09 173018 A (KYODO NYUGYO KK), 8 July 1997 (1997-07-08)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to infant formulas or baby food supplemented with osteopontin and use of osteopontin in infant formulas or baby food.

BACKGROUND OF THE INVENTION

It is well known that infant formulas do not possess the same immune stimulating effect as mother milk. Therefore breast fed babies have less infections together with less development of allergy and eczema compared to bottle fed babies. For this reason infant formulas are attempted to resemble human milk as much as possible. This is called to "humanize" infant formulas.

Osteopontin (OPN) is a protein present in milk from milk producing mammals, e.g. human and bovine milk.

So far it has been believed that human milk and bovine milk contained OPN in nearly the same concentration. Therefore it has not been proposed to add extra OPN to infant formulas in order to humanize infant formulas.

The content of OPN in human milk was estimated to be about 3-10 mg/litre. Surprisingly it has now been found that human milk on an average contains about 25 - 300 mg/litre, i.e. about 5 - 10 times as much OPN as bovine milk. Individual differences occur.

It has also been shown that OPN possesses a key function in the acquisition of Th-1-response. Infants possess from birth mainly Th-2 responses, due to the antibodies received from the mother and the maturation of the immune response in infants is mediated by the induction/acquisition of Th-1 responses. OPN is therefore believed to be an essential component in human milk for infant nutrition.

OPN is a multifunctional protein, involved in both physiological and pathological processes in multiple organs and tissues including biomineralization, inflammation, leukocyte recruitment and cell survival (reviewed in; Mazzali M, Kipari T, Ophascharoensuk V, Wesson JA, Johnson R, Hughes J.2002.Osteopontin - a molecule for all reasons. Q.J. 95:3-13; Denhart DT, Giachelli CM, Rittling SR.2001. Role of osteopontin in cellular signaling and toxicant injury. 41: 4723-49). OPN is expressed by a number of cell types and epithelial cells. Accordingly, OPN is present in most tissues and organs that have been analyzed for its presence. OPN is present in relatively high concentrations in many body fluids, such as blood, plasma, bile, urine and milk. OPN exists in tissue-specific isoforms, e.g. proteolytically truncated forms, phosphorylation- and glycosylation variants etc., which may correspond to particular cellular functions.

Bovine milk OPN, the most well characterized form of the protein, contains 27 phosphoserines, one phosphothreonine and three O-glycosylated threonines (Sørensen ES, Højrup P, Petersen T. 1995. Posttranslational modifications of bovine osteopontin: Identification of twenty-eight phosphorylation and three O-glycosylations sites. Protein Sci. 4:2040-2049). All phosphorylations are situated in acidic recognition sequences of the mammary gland casein kinase and casein kinase 2 (Sørensen ES, Petersen TE. 1994. Identification of two phosphorylation motifs in bovine osteopontin. Biochem.Biophys.Res.Comm.198:200-205).

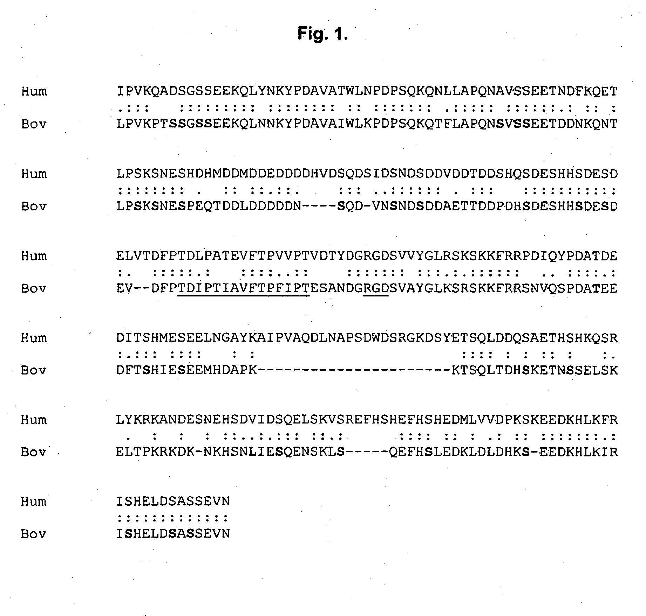

Bovine and human OPN are very homologous with all functional elements being retained among the species. An alignment of the two proteins is shown in Fig 1. The Arg-Gly-Asp integrin binding sequence is responsible for binding to cell receptors, the poly aspartic acid rich region is involved in the binding to hydroxyapatite, calcium salt and ions, and most interestingly almost all serine residues located in the recognition sequence of the mammary gland casein kinase shown to phosphorylate the bovine OPN is also present in the human sequence. Likewise, the threonine residues shown to be glycosylated in the bovine protein is also present in the human counterpart. This preservation of sites and motifs of posttranslational modification indicates that bioactive peptides that can be formed by digestion of the human OPN are most likely also formed by digestion of the bovine OPN. Therefore, the bovine protein is believed to be highly suitable to fortify e.g. infant formulas and baby food.

WO 02/28413 discloses the purification of an acidic protein fraction of milk protein by anion-exchange and the use of this fraction in bone health compositions. The described fraction contains a number of minor acidic whey proteins. These include proteose peptone component 3, proteose peptone component 5 (PP5), also known as beta-casein-5P (fl-1 05) or beta-casein-5P (fl-107), proteose peptone 8-slow (PP8-slow), also known as beta-casein-1 P (f29-105) or beta-casein-1P (f29-107), sialyated and phosphorylated proteins alpha-s1-casein phosphopeptides and osteopontin, and also a mixture of peptides derived from these proteins as well as small amounts of betalactoglobulin, bovine serum albumin and immunoglobulins. The protein fraction described in the invention may be used for the generation of functional foods for the treatment and/or prevention of bone defects. The patent specification does not identify osteopontin as being the active component in the protein fraction, and the patent specification does not mention the use of osteopontin or the protein fraction described for immune stimulating purposes or for humanization of infant formulas.

Senger DR, Perruzzi CA, Papadopoulos A, Tenen DG. 1989. Biochem.Biophys.Acta. 996: 43-48. Purification of a human milk protein closely similar to tumor secreted phosphoproteins and osteopontin, describes the presence and purification of osteopontin in human breast milk. The authors estimate the concentration of osteopontin in human milk to be in the range of 3 -10 mg/litre. This estimate is more than 10-fold below the value we measure in the present invention.

Dhanireddy R, Senger R, Mukherjee BB, Mukherjee AB. 1993. Acta Paediatr. 82:821-2. Osteopontin in human milk from mothers of premature infants, describes the presence of osteopontin in preterm milk. The milk samples are characterized by Western blotting. The concentrations of OPN in the samples are not determined. The presence of OPN in milk is only discussed in relation to calcium transport.

Sorensen S, Justesen SJ, Johnsen, AH. 2003. Protein Expression and Purification 30:238-245. Purification and characterization of osteopontin from human milk, describes a new protocol for purification of human milk osteopontin. The article describes the purification of several fragments and peptides derived from osteopontin as well as intact full-length osteopontin. The estimation of the concentration of osteopontin in human milk is based on the amount purified by the procedure, and is roughly in agreement with the findings of Senger et al. 1989. This is more than 10-fold below the osteopontin value we measure by sandwich ELISA in the present invention. The article also describes the production of polyclonal antibodies against osteopontin, but these are not used to determine the actual amount of osteopontin in milk. The article simply describes a way of preparing osteopontin and osteopontin variants for standardization procedures, antibody production and functional studies. The article does not discuss or mention the function of, or potential use of osteopontin in relation to milk, infant formulas or any other food product.

Choi, K. W. et al in "Purification and properties of osteopontin from bovine milk" (June 2003) (XP002281089 database accession no. PREV200300375294) discloses bovine milk, containing 31.7-39.7 mg osteopontin/liter.

Bayless, K.J. et al in "Isolation and Biological Properties of Osteopontin from Bovine Milk", Protein expression and purification, vol. 9, no. 3 (April 1997), pages 309-314, discloses bovine milk containing 8 mg osteopontin/liter.

WO 01/11990 discloses an infant formula.

Assays determining the OPN concentration in prior art estimates the content in the range of 3 - 10 mg/litre. However assays determining the OPN concentration in milk are difficult to perform due to, e.g. the interference of proteins forming aggregates. Therefore the OPN content in milk has not been determined precisely. Due to this OPN has not been added to infant formulas, in order to obtain a product which resembles mother milk with the beneficial protective effect against the development of allergy. Therefore infant formulas are currently not provided with immune stimulatory factors, which can increase the acquisition of Th1 responses.

SUMMARY OF THE INVENTION

The invention is based on the surprising discovery that OPN is present in elevated levels in human breast milk compared to bovine milk. Previous estimations of the OPN content in milk have been based on Western blotting analyses and protein purification, i.e. 3 -10 mg/litre. The development of a quantitative ELISA directed against milk OPN has lead to new insight into the concentration of OPN in milk.

Accordingly the present invention provides infant formulas or baby food supplemented with milk osteopontin of DL non-human origin, wherein the milk osteopontin is contained in an amount which results in at least 50-300 MG osteopontin/litre in a ready to feed formula. In all known infant formulas OPN can be included as supplement or OPN can be given to the infant separately from the formula which promote acquisition of Th1 response and thus reduce impaired immune function in infants.

The amount of osteopontin given to the infant, either in an infant formula or separately from an infant formula can be comparable to that found in human breast milk. The osteopontin may be incorporated into all human infant formulas, such as bovine milk based and soy based infant formulas and in baby food such as NAN® from Nestlé.

Infant formulas manufactured as starter formulas, follow-on formulas and LBW formulas can all comprise milk OPN.

Infant formulas should preferably comprise milk OPN in an amount of at least 1 % of the total protein content corresponding to the amount of milk OPN in human breast milk.

BRIEF DESCRIPTION OF FIGURES

Figure 1 shows an alignment of bovine and human milk OPN. Phosphorylated residues in bovine OPN are shown in bold letters.

Figure 2 shows IL-12 expression in human intestinal T cell after stimulation with bovine OPN.

DETAILED DESCRIPTION OF PREFERRED EMBODIMENTS

In a large number of newborn infants who are not nursed by the mother but fed by an infant formula the acquisition of Th1 immune response may be delayed, thereby increasing the risk of allergy reactions. By giving the non-nursed new-born infant formulas supplemented with milk OPN, in amounts comparable to human milk, the induction of Th1 immune response is likely to occur.

Thus the invention proposes an infant formula or baby food supplemented with osteopontin.

Infant formulas preferably comprise osteopontin obtained from DL bovine milk. However, infant formulas can also be supplemented with osteopontin from milks of other animals such as goat, sheep, camel, dromedary or llama.

Preferably infant formulas comprise osteopontin in an amount corresponding to 1% of the total protein content. As much as 3% can be used, and preferably used amounts are more than 1.5%, 2% or 2.5%. The amount of milk osteopontin in the ready to feed formula is 50 - 300 mg/litre, preferably 100 - 200 mg/litre. 130 - 150 mg/litre is most preferred. In many cases 130 mg/litre is believed to give the best infant formulas.

Another aspect of the present invention is the use of milk osteopontin as supplement in infant formulas or baby food.

Also certain immune deficiencies are believed to be treated by giving formulas with an extra supplement of milk osteopontin.

Milk osteopontin can as mentioned be obtained from all mammalian, such as camel, goat, sheep, llama and dromedary. Osteopontin is preferably obtained by purification from DL bovine milk.

Osteopontin can be applied to all known infant formulas, i.e. starter formulas, follow on formulas and LBW-formulas and premature formulas.

The concentration of milk osteopontin can depend on geographical position, ethnical group, lifestyle, food etc.

Milk osteopontin can also be applied to infant formulas given to other mammals than humans, such as domesticated animals, livestocks (pigs, cows, horses, etc.) pets and zoo animals.

Milk taken at different day's post-partum can be used as a measurement for the osteopontin concentration in human breast milk at different times post partum. This measurement can be used to have different OPN levels in, e.g. starter formulas; follow on formulas and LBW-formulas.

It is also possible to measure the OPN content in a mother's milk and supplement it with extra milk OPN if necessary and give it to her own child or another child.

The measurement of OPN in human milk may be carried out using ELISA or other immunological procedures such as Western blotting analyses.

An ELISA can be carried out in a number of different embodiments, many of which are applicable in the present invention. One ELISA embodiment, which is particularly suitable for use in the present invention, use antibodies specific for native milk OPN.

The infant formulas of the present invention are supplemented with milk OPN isolated from milk, however it will be appreciated that milk OPN may be prepared by recombinant means in appropriate cells yielding a phosphorylation pattern as found in the 'milk-OPN'. Preferred cells for expression are those of the mammary gland, since these cells may be expected to yield an identical or essentially identical phosphorylation pattern.

It is well know that infant formulas do not possess the same immune stimulating effect as mother milk and breast fed babies are having less infections together with development of allergy and eczema compared to bottle fed babies. One reason could be that breast milk is containing high amounts of OPN.

Definitions

As used herein the term "osteopontin" or "OPN" is used for osteopontin from milk, including naturally occurring fragments or peptides derived from OPN by proteolytic cleavage in the milk, or splice-, phosphorylation-, or glycosylation variants as obtainable from the method proposed in WO 01/49741.

Milk OPN is OPN purified and obtained from mammalian milk samples.

Mammalian milk is milk from DL any non-human milk producing animals, such as cows, camels, goats, sheep, dromedaries and llamas.

Breast milk: Milk collected from (healthy) human mothers.

Infant formulas are formulas which cover the nutrition need of an infant.

Starter formulas: formulas for babies which cover their nutrition needs within the first 4-6 months.

Follow-on formulas: formulas for babies which cover their nutrition needs after the first 4 months.

LBW (low birth weight) formulas: Formulas which cover the nutrition needs of an infant with low birth weight.

Premature formulas: Formulas which cover the nutrition needs of a premature infant.

The invention is further illustrated referring to the following non-limiting examples.

**Examples**

Collection and treatment of milk samples

Human breast milk samples were collected from 10 healthy mothers 10-58 days post partum. The milk was collected by breast pump and 2 ml were sampled from each milking and pooled to obtain whole day milk. The cream was extracted from whole milk by high speed centrifugation and separated from skimmed milk. The skimmed milk was aliquoted into 0.2 ml fractions and analyzed immediately or frozen at -20°C until required.
Fresh pooled cow's milk was obtained at a local dairy. Milk was obtained four times over a period of 4 months and processed individually. The cream was removed by centrifugation, and samples of the milk were frozen at -20°C until required.

Measurement of total protein concentration in milk

The protein concentration in all milk samples was determined by Bradford analysis using bovine serum albumin as standard. Analyses were performed according to the descriptions supplied by the manufacturer.

ELISA for OPN in milk

The concentration of OPN in the individual milks and in commercially available infant formulas was measured by a sandwich ELISA. For this assay, antiserum against bovine and human OPN against native OPN purified from bovine and human milk, respectively, were raised in rabbits at Dako (Glostrup, Denmark). The bovine OPN was purified essentially as described (Sorensen ES, Petersen TE, 1993, Purification and characterization of three proteins isolated from the proteose peptone fraction  of bovine milk, J.Dairy Res. 60:189-197). Human milk OPN was purified essentially as described (Senger DR, Perruzzi CA, Papadopoulos A, Tenen DG, 1989, Purification of a milk protein closely similar to tumor-secreted phosphoproteins and osteopontin. Biochem.Biophys.Acta 996:43-48). For extra high purity both proteins were subjected to reverse-phase chromatography before immunization of the rabbits. The IgG fraction of the antiserum was purified by Concavalin A affinity and used directly in the ELISA, or purified on an OPN affinity column to obtain highly specific antibodies. The specificities of the antibodies were checked and verified by Western blotting analyses of milk samples and purified OPN. The developed ELISA was very sensitive with a detection limit <5ng/ml milk, and linearity in the range of 10-300 ng/ml, accordingly milk samples were diluted 2000-23000 times before analyses. All samples were analyzed in triplicates in 6-12 dilutions per measurement. The reliability of the ELISA was checked by spiking milk samples with the known amounts of OPN showing that the assay was quantitative with full recovery of the spiked amount of OPN in the range used for these measurements. Likewise, purification of OPN from human milk samples showed OPN yield comparable with the concentrations measured by the ELISA.

Concentration of OPN in bovine milk

The level of OPN in pooled bovine milk was determined four times during a 4 months period (Table 1). The average OPN concentration was found to be 18.28 mg/litre. The total protein was set to 35,000 mg/litre according to the literature. The OPN content in bovine milk is 0.05% (w/w) of the total protein.

Table 1. OPN concentration in bovine milk

| Bovine milk 'sample no.' | OPN (mg/litre) | Total protein (mg/litre) | OPN/Protein (%) |
|---|---|---|---|
| 301002 | 17.84 | | |
| 061102 | 19.37 | | |
| 141101 | 17.91 | | |
| 120203 | 18.01 | | |
| Average | 18.28 | ~35,000 | 0.052 |

Concentration of OPN in human milk

The milk from 10 mothers, aged 25-35 years (average 29 years) was sampled 10-58 days post partum (average 21.6 days post-partum) and analyzed for OPN and total protein. The data are summarized in table 2. The OPN in the breast milk ranged from 22.44 mg/litre to 257.35 mg/litre with an average value of 138.5 mg/litre. The total protein concentration in the milk ranged from 8327 mg/litre to 13268 mg/litre with an average value of 10821 mg/litre. The OPN contributes an average of 1.3% (w/w) of the total protein in human milk and in a single mother more than 3% of the milk protein was OPN.

Table 2. OPN concentration in human milk.

|  | Age of mother | Days post partum | OPN Concentration (mg/litre) | Total protein concentration (mg/litre) | OPN protein (%) |
|---|---|---|---|---|---|
|  | 28 | 59 | 65.97 | 8550 | 0.77 |
|  | 35 | 13 | 257.35 | 8327 | 3.09 |
|  | 28 | 12 | 200.52 | 13268 | 1.50 |
|  | 30 | 10 | 238.69 | 12628 | 1.89 |
|  | 25 | 12 | 22.44 | 11442 | 0.20 |
|  | 30 | 33 | 101.54 | 10000 | 1.02 |
|  | 31 | 39 | 113.96 | 10157 | 1.12 |
|  | 30 | 17 | 67.04 | 10328 | 0.65 |
|  | 23 | 12 | 212.00 | 10936 | 1.94 |
|  | 30 | 10 | 105.00 | 12576 | 0.83 |
| Average | 29 | 21.6 | 138.4 | 10821 | 1.3 |

There is a considerable variation in the OPN concentration related to total protein in individual mothers. In all mothers the OPN/total protein ratio was significantly higher than the corresponding value for bovine milk. These measurements show that the average OPN concentration related to total protein in human milk (1.3%) compared to bovine milk (0.05%) is about 26 times higher. This average value covers individual variations in the range 4 to 62 times the bovine value.

Concentration of OPN in commercially available infant formulas

Data are calculated as mg OPN/liter ready to feed formula, based on 125 g/liter.

Table 3

| From the firm | Infant Formula | Content of OPN, mg/liter |
|---|---|---|
| Nam Yang | Science 1 | 11.5 |
|  | Science 2 | 9.4 |
|  | Agisarang 3 | 12.3 |
|  | Agisarang 4 | 17.4 |
|  | XO1 | 7.9 |
|  | XO2 | 6.2 |
|  | XO3 | 8.4 |
|  | XO4 | 6.7 |
|  | Agisarang soo | 5.5 |
|  | Imperial Dream | 6.0 |
|  | Science | 17.8 |
|  | Premium XO | 13.0 |

(continued)

| From the firm | Infant Formula | Content of OPN, mg/liter |
|---|---|---|
| Nestlé | Nan 1 | 10.3 |
| | Nidina 1 | 5.3 |
| Beauvais | Allomin 2 | 6.4 |

Bovine osteopontin induces IL-12 expression in human intestinal gut cells

Human intestinal cells were obtained and cultured in the presence of IL-2 and IL-4 essentially as described (Agnholt J, Kaltoft K, 2001. Infliximab downregulates interferon production in activated gut T-lymfocytes from patients with Crohn's disease. Cytokine.15:212-222). The cultivation in the presence of IL-2 and IL-4 promote the growth of T-cells with preserved cellular features such as expression of the receptors; TCR$\alpha\beta$+, CD4+CD45RO+.

Cell culture wells were coated with 1 ml (1mg/ml) osteopontin PBS-solution and PBS, respectively, over night at 4°C. The wells were emptied and T-cells were cultured in the wells ($10^6$ cells/ml) for 24 hours and subsequently aliquots of the cell supernatants were sampled for analyses.

Cytokine-matched antibody pairs for determination of IL-12 were obtained from R&D Systems. The detecting antibodies were all biotinylated. A time-resolved fluorometric assay applying Europium ($Eu^{3+}$) labelled streptavidin and a Delphia 1234 fluorometer (Wallac, Turku, Finland) were used to determine the IL-12 content. Obtained values are averages of three ELISA readings in triplicate experiments.

Table 4. IL-12 expression in human gut immune after stimulation with bovine osteopontin

| | IL-12 (pg/ml) |
|---|---|
| | Average (std. dev.) |
| Control cells | 3.3 (+/- 3.2) |
| + Osteopontin | 16.7 (+/-2.1) |

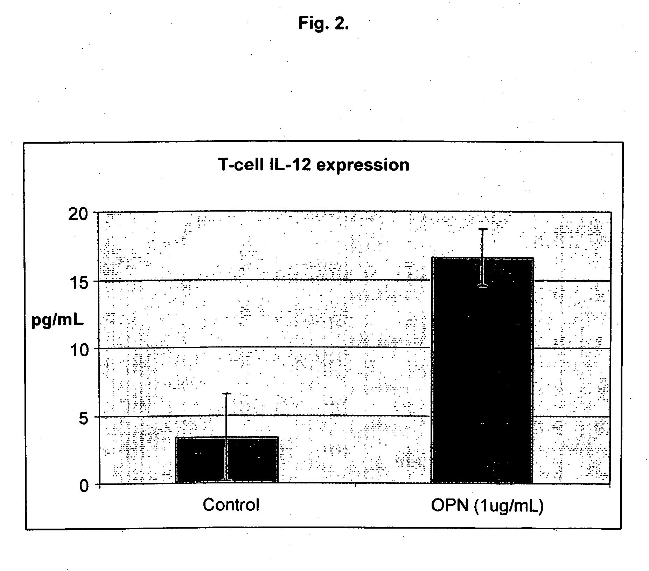

As shown in table 4 and figure 2, a significant raise in the expression of IL-12 can be observed in the human immune cells stimulated with native bovine osteopontin.

Here we show that native bovine OPN is able to induce expression of the Th-1 cytokine IL-12 in non-activated human intestinal immune cells, strongly indicating a role in the regulation of intestinal immunity.

Mix of infant formulas comprising OPN

A level of 100 - 130 mg/litre OPN is considered a suitable amount for infant formulas in the following examples. However, the scope of the present invention is not limited to this amount since variation in human milk compositions due to food, lifestyle, geographical area, ethnical group etc exist.

Incorporated in the examples of the present invention are infant formula compositions, which have been prepared, based on EU legislation and recommendations for infant formulas.

In connection with this we only take the protein amount in account, since the other parts of the product are expected to be unchanged. We have chosen to apply 100 mg OPN/1000 ml to all products, however it is also possible and within the scope of the present invention to apply OPN according to the protein level per se.

Cow milk contains OPN in the range of 15 - 20 mg/litre which is at least 5 times below the OPN content in human milk measured in the examples in this invention to an average of 138.4 mg/litre. OPN in the range of 15 - 20 mg/litre corresponds to 0.05% of the total protein content in bovine milk. This figure is approximately 26 times lower than 1.3% (OPN/total protein) as measured in human milk in the examples in this invention.

We have in our calculations assumed that OPN is contained in the whey part of the milk and the level will therefore be approximately 18 mg/100 g milk powder and approximately 360 mg/100 g WPC80 powder. Due to these numbers we have calculated the level, which is expected to arise from the used ingredients.

In some of the following examples we have reduced the part of whey protein by the additional OPN amount, since the glycoprotein is considered to follow whey under the precipitation of rennet-casein at approximately pH 6,2 and acid casein at pH 4,6.

The protein level for infant formulas can be calculated as follows:

Starter formulas

Starter formulas mean food for babies which cover their nutrition needs within the first 4-6 months.

Regulatory requirements for the protein content within EU.

Source: Announcement no. 202 regarding infant formulas and supplements for newborn infants and babies:

Proteins:

$$\text{Protein content} = \text{nitrogen content} \times 6.38 \text{ when cow milk proteins}$$

$$\text{Protein content} = \text{nitrogen content} \times 6.25 \text{ when soy milk proteins isolator and partly hydrolyzed protein.}$$

Protein content = nitrogen content x 6.25 when soy milk proteins isolator and partly hydrolyzed protein.

By chemical index is meant the lowest ratios between the amount of essential amino acid in the present protein and the amount of the corresponding amino acid in the reference protein.

Products based on cow milk:

| | Minimum | Maximum |
|---|---|---|
| Starter formulas | 0.45 g/100 kJ (1.8 g/100 kcal) | 0.7 g/100 kJ (3 g/100 kcal) |

At the same energy content the product should contain an accessible amount of essential and semi essential amino acids as the reference protein (mother milk as defined in annex 6); at the calculation the methionin and cystein content can be added.

Products based on partly hydrolyzed protein:

| | Minimum | Maximum |
|---|---|---|
| Starter formulas | 0.56 g/100 kJ (2.25 g/100 kcal) | 0.7 g/100 kJ (3 g/100 kcal) |

At the same energy content the product should contain an accessible amount of essential and semi essential amino acids as the reference protein (mother milk as defined in annex 6); at the calculation the methionin and cystein content can be added.

Protein effectiveness ratio (PER) and netto protein utilization (NPU) should correlate at least to casein. Taurin content has to be at least 10 $\mu$mol/100 kJ (42 $\mu$mol/100 kcal) and the L-carnithin content should at least be 1.8 $\mu$mol 100 kJ (7.5 $\mu$mol/100 kcal).

Products based solely on soy protein isolates or in compositions together with cow milk proteins:

| | Minimum | Maximum |
|---|---|---|
| Starter formulas | 0.56 g/100 kJ (2.25 g/100 kcal) | 0.7 g/100 kJ (3 g/100 kcal) |

Only soy protein isolates can be used for these infant formulas. The chemical index of the proteins should be at least 80 percent of the reference protein (mother milk defined in annex 7 in the announcement).

At the same energy content the product should contain at least an accessible amount of methionin comparable to the reference protein (mother milk). The content of L-camithin should be at least 1.8 $\mu$mol/100 kJ (7.5 $\mu$mol/100 kcal).

Amino acids can only be applied to products in order to enhance the nutritional value and only in ratios, necessary to reach the aim.

Examples: mix for starter formulas

Energy: 2200 kJ/100 g/525 kcal/100 g)

Reconstitution: 125 g per litre

Traditional infant starter formula

|  | Powder gram per 100 g | Reconstituted gram per litre | Gram per 100 kcal | Gram per 100 kJ |
|---|---|---|---|---|
| Total protein * | 12 | 15 | 2.29 | 0.550 |
| Casein | 4.8 | 6.1 | 0.91 | 0.220 |
| Whey protein | 7.2 | 9.1 | 1.38 | 0.330 |
| *of this OPN | 0.030 | 0.038 | 0.006 | 0.001 |
| Ratio Casein/Whey protein | 40/60 |  |  |  |

Infant starter formula comprising OPN

|  | Powder gram per 100 g | Reconstituted gram per litre | Gram per 100 kcal | Gram per 100 kJ |
|---|---|---|---|---|
| Total protein | 12 | 15 | 2.29 | 0.550 |
| Casein | 4.80 | 6.1 | 0.91 | 0.220 |
| *of this OPN | 0.030 | 0.038 | 0.006 | 0.001 |
| Whey protein | 7.12 | 9.0 | 1.36 | 0.326 |
| Added OPN | 0.074 | 0.092 | 0.014 | 0.003 |
| Ratio casein/ whey protein | 40/60 |  |  |  |

Follow-on formulas

Regulatory requirements for the protein content within EU.

Source: Announcement no. 202 regarding infant formulas and supplements for newborn infants and babies:

Proteins:

Protein content = nitrogen content x 6.38 when cow milk proteins

Protein content = nitrogen content x 6.25 when soy milk proteins isolator

By chemical index is meant the lowest ratios between the amount of essential amino acid in the present protein and the amount of the corresponding amino acid in the reference protein.

Products based on cow milk:

|  | Minimum | Maximum |
|---|---|---|
| [Follow-on formulas] | 0.50 g/100 kJ (2.25 g/100 kcal) | 1 g/100 kJ (4.5 g/100 kcal) |

The chemical index of the proteins should be at least 80 percent of the reference protein (mother milk defined in annex 7 in the announcement). Only soy protein isolates can be used for these infant formulas. Amino acids can only be applied to products in order to enhance the nutritional value and only in ratios necessary to reach the aim.

At the same energy content the product should contain an accessible amount of essential and semi essential amino acids as the reference protein (mother milk as defined in annex 6); at the calculation the methionin and cystein content can be added.

Examples:

> Energy: 2170 kJ/100g (520 kcal/100g)
> Reconstitution: 150 g per litre

Traditional follow-on formulas

|  | Powder gram per 100 g | Reconstituted gram per litre | Gram per 100 kcal | Gram per 100 kJ |
|---|---|---|---|---|
| Total protein * | 15 | 22.5 | 2.89 | 0.690 |
| Casein | 12 | 18 | 2.31 | 0.554 |
| Whey protein | 3 | 4.5 | 0.58 | 0.138 |
| *of this OPN | 0.007 | 0.011 | 0.001 | 0.000 |
| Ratio casein/whey protein | 80/20 |  |  |  |

Follow-on formula comprising OPN

|  | Powder gram per 100 g | Reconstituted gram per litre | Gram per 100 kcal | Gram per 100 kJ |
|---|---|---|---|---|
| Total protein | 15 | 22.5 | 2.89 | 0.690 |
| Casein | 12 | 18 | 2.31 | 0.554 |
| Whey protein | 2.92 | 4.4 | 0.56 | 0.134 |
| *of this OPN | 0.007 | 0.011 | 0.001 | 0.000 |

(continued)

|  | Powder gram per 100 g | Reconstituted gram per litre | Gram per 100 kcal | Gram per 100 kJ |
|---|---|---|---|---|
| OPN | 0.078 | 0.117 | 0.015 | 0.004 |
| Ratio Casein/Whey protein | 80/20 |  |  |  |

Infant formulas for Low-Birth weight (LBW) babies

Presently there is no legislation in this area, however it is under process. Manufacturers of infant formulas for LBW babies are currently getting recommendations from pediatrists and "IDACE proposal for guidelines on compositions of low-birth-weight formulae for marketing in the European Union". These guidelines are expected to provide the base for the upcoming legislation in EU.

Proteins:

Protein content = nitrogen x 6.38 for cow milk and hydrolyzed cow milk proteins.

Products based on cow milk and hydrolyzed cow milk proteins.

|  | Minimum | Maximum |
|---|---|---|
| Low-birth-weight | 0.6 g/100 kJ (2.4 g/100 kcal | 0.8 g/100 kJ (3.3 g/100 kcal) |

The formulas have to contain an accessible amount of each essential and semi essential amino acid at least comparable to the content in the reference protein (breast milk). In contrast to standard infant formulas the amount of methionin and cystein cannot be added for calculation purposes The taurine content should be at least 1.3 mg/100 kJ (5.3 mg/100 kcal).

Example

Energy: 315 kJ / 100 ml (75 kcal/100 ml)
Reconstitution: 150 g per litre

Traditional LBW formula

|  | Powder gram per 100 g | Reconstituted gram per litre | Gram pr. 100 kcal | Gram pr. 100 kJ |
|---|---|---|---|---|
| Total protein * | 15 | 22.5 | 3 | 0.718 |
| Hydrolyzed whey protein | 14.96 | 22.44 | 2.992 | 0.716 |
| *of this OPN | 0.067 | 0.101 | 0.012 | 0.002 |
| Taurine | 0.04 | 0.06 | 0.008 | 0002 |

LBW formula comprising OPN:

|  | Powder gram per 100 g | Reconstituted gram per litre | Gram per 100 kcal | Gram per 100 kJ |
|---|---|---|---|---|
| Total protein * | 15 | 22.5 | 3.000 | 0.718 |
| Hydrolyzed whey protein | 14.89 | 22.34 | 2.972 | 0.712 |
| *of this OPN | 0.067 | 0.101 | 0.012 | 0.002 |
| Taurine | 0.04 | 0.06 | 0.008 | 0.002 |
| OPN added | 0.023 | 0,034 | 0,005 | 0,002 |

**Figures**

Fig. 1.
Alignment of human and bovine OPN. Identity is indicated by two dots and homologous amino acids are indicated by a single dot. Phosphorylated residues in bovine OPN are shown in bold. The RGD (Arg-Gly-Asp) integrin binding triplet and the region containing the glycosylations in the bovine sequence are underlined

Fig. 2.
IL-12 expression of T-cells stimulated with bovine OPN.

**Claims**

1. Infant formula or baby food supplemented with milk osteopontin of non-human mammalian origin, wherein the milk osteopontin is contained in an amount which results in at least 50-300 mg osteopontin /litre in a ready to feed formula.

2. Infant formula or baby food according to claim 1 wherein milk osteopontin is obtained from bovine milk.

3. Infant formula or baby food according to any of the preceding claims wherein the milk osteopontin is contained in an amount which results in at least 50-250 mg osteopontin /litre in the ready to feed formula.

4. Infant formula or baby food according to claim 3 wherein the milk osteopontin is contained in an amount which results in at least 100-200 mg osteopontin /litre in the ready to feed formula.

5. Infant formula or baby food according to claim 4 wherein the milk osteopontin is contained in an amount which results in at least 130-150 mg osteopontin /litre in the ready to feed formula.

6. Infant formula or baby food according to any of the preceding claims wherein the infant formula is a starter formula.

7. Infant formula or baby food according to any of the preceding claims wherein the infant formula is a follow-on formula.

8. Use of milk osteopontin as supplement in infant formulas or baby food.

9. Use according to claim 8 wherein milk osteopontin is obtained from human or bovine milk.

10. Use according to any of the claims 8 to 9 wherein milk osteopontin is contained in an amount which results in at least 50-300 mg osteopontin /litre in the ready to feed formula.

11. Use according to any of the claims 8 to 10 wherein the infant formula is a starter formula.

**12.** Use according to any of the claims 9 to 12 wherein the infant formula is a follow-on formula.

**Patentansprüche**

**1.** Säuglingsanfangsnahrung oder Babynahrung, welche mit aus nichtmenschlichen Säugetieren gewonnenem Milch-Osteopontin ergänzt ist, wobei das Milch-Osteopontin in einer Menge, die zu mindestens 50-300 mg Osteopontin/ Liter in einer Fertignahrung führt, enthalten ist.

**2.** Säuglingsanfangsnahrung oder Babynahrung nach Anspruch 1, wobei das Milch-Osteopontin aus Kuhmilch gewonnen ist.

**3.** Säuglingsanfangsnahrung oder Babynahrung nach einem der vorgehenden Ansprüche, wobei das Milch-Osteopontin in einer Menge, die zu mindestens 50-250 mg Osteopontin/Liter in der Fertignahrung führt, enthalten ist.

**4.** Säuglingsanfangsnahrung oder Babynahrung nach Anspruch 3, wobei das Milch-Osteopontin in einer Menge, die zu mindestens 100-200 mg Osteopontin/Liter in der Fertignahrung führt, enthalten ist.

**5.** Säuglingsanfangsnahrung oder Babynahrung nach Anspruch 4, wobei das Milch-Osteopontin in einer Menge, die zu mindestens 130-150 mg Osteopontin/Liter in der Fertignahrung führt, enthalten ist.

**6.** Säuglingsanfangsnahrung oder Babynahrung nach einem der vorgehenden Ansprüche, wobei die Säuglingsanfangsnahrung eine Anfangsnahrung ist.

**7.** Säuglingsanfangsnahrung oder Babynahrung nach einem der vorgehenden Ansprüche, wobei die Säuglingsanfangsnahrung eine Folgenahrung ist.

**8.** Verwendung von Milch-Osteopontin als Ergänzung bei Säuglingsanfangsnahrungen oder Babynahrung.

**9.** Verwendung nach Anspruch 8, wobei das Milch-Osteopontin aus Muttermilch oder Kuhmilch gewonnen ist.

**10.** Verwendung nach einem der Ansprüche 8 bis 9, wobei das Milch-Osteopontin in einer Menge, die zu mindestens 50-300 mg Osteopontin/Liter in der Fertignahrung führt, enthalten ist.

**11.** Verwendung nach einem der Ansprüche 8 bis 10, wobei die Säuglingsanfangsnahrung eine Anfangsnahrung ist.

**12.** Verwendung nach einem der Ansprüche 9 bis 12, wobei die Säuglingsanfangsnahrung eine Folgenahrung ist.


**Revendications**

**1.** Formule pour nourrissons ou alimentation pour bébés complété avec l'ostéopontine de lait d'origine mammifère non humaine, l'ostéopontine de lait étant contenue dans une quantité qui aboutit à au moins 50 à 300 mg/litre d'ostéopontine dans une formule prête à alimenter.

**2.** Formule pour nourrissons ou alimentation pour bébés selon la revendication 1, où l'ostéopontine de lait est obtenue à partir du lait bovin.

**3.** Formule pour nourrissons ou alimentation pour bébés selon l'une quelconque des revendications précédentes, où l'ostéopontine de lait est contenue dans une quantité qui aboutit à au moins 50 à 250 mg/litre d'ostéopontine dans la formule prête à alimenter.

**4.** Formule pour nourrissons ou alimentation pour bébés selon la revendication 3, où l'ostéopontine de lait est contenue dans une quantité qui aboutit à au moins 100 à 200 mg/litre d'ostéopontine dans la formule prête à alimenter.

**5.** Formule pour nourrissons ou alimentation pour bébés selon la revendication 4, où l'ostéopontine de lait est contenue dans une quantité qui aboutit à au moins 130 à 150 mg/litre d'ostéopontine dans la formule prête à alimenter.

6. Formule pour nourrissons ou alimentation pour bébés selon l'une quelconque des revendications précédentes, où la formule pour nourrissons est une formule d'entrée.

7. Formule pour nourrissons ou alimentation pour bébés selon l'une quelconque des revendications précédentes, où la formule pour nourrissons est une formule de suivi.

8. Utilisation de l'ostéopontine de lait en tant qu'un complément dans les formules pour nourrissons ou alimentations pour bébés.

9. Utilisation selon la revendication 8, où l'ostéopontine de lait est obtenue à partir du lait humain ou bovin.

10. Utilisation selon l'une quelconque des revendications 8 à 9, où l'ostéopontine de lait est contenue dans une quantité qui aboutit à au moins 50 à 300 mg/litre d'ostéopontine dans la formule prête à alimenter.

11. Utilisation selon l'une quelconque des revendications 8 à 10, où la formule pour nourrissons est une formule d'entrée.

12. Utilisation selon l'une quelconque des revendications 9 à 12, où la formule pour nourrissons est une formule de suivi.

EP 1 667 537 B1

**Fig. 1.**

```
Hum    IPVKQADSGSSEEKQLYNKYPDAVATWLNPDPSQKQNLLAPQNAVSSEETNDFKQET
       .::: :::::::::: :::::::: :: :::::::: .::::: :::::::.: :: :
Bov    LPVKPTSSGSSEEKQLNNKYPDAVAIWLKPDPSQKQTFLAPQNSVSSEETDDNKQNT


Hum    LPSKSNESHDHMDDMDDEDDDDHVDSQDSIDSNDSDDVDDTDDSHQSDESHHSDESD
       ::::::::: . :: ::..::. ::: ..::::::: . ::: ::::::::::::::
Bov    LPSKSNESPEQTDDLDDDDDDN----SQD-VNSNDSDDAETTDDPDHSDESHHSDESD


Hum    ELVTDFPTDLPATEVFTPVVPTVDTYDGRGDSVVYGLRSKSKKFRRPDIQYPDATDE
       :. :::::.: :::::..:: :::::::: :::.:.::::::: .. ::::..:
Bov    EV--DFPTDIPTIAVFTPFIPTESANDGRGDSVAYGLKSRSKKFRRSNVQSPDATEE


Hum    DITSHMESEELNGAYKAIPVAQDLNAPSDWDSRGKDSYETSQLDDQSAETHSHKQSR
       :.::: :::: : : :::: : : :: : :.
Bov    DFTSHIESEEMHDAPK-------------------KTSQLTDHSKETNSSELSK


Hum    LYKRKANDESNEHSDVIDSQELSKVSREFHSHEFHSHEDMLVVDPKSKEEDKHLKFR
       . : : : ::..:.::: ::.:. :::: :: : .: :: ::::::::.:
Bov    ELTPKRKDK-NKHSNLIESQENSKLS-----QEFHSLEDKLDLDHKS-EEDKHLKIR


Hum    ISHELDSASSEVN
       :::::::::::::
Bov    ISHELDSASSEVN
```

15

**Fig. 2.**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0228413 A
- WO 0111990 A

- WO 0149741 A

### Non-patent literature cited in the description

- **MAZZALI M ; KIPARI T ; OPHASCHAROENSUK V ; WESSON JA ; JOHNSON R ; HUGHES J.** *Osteopontin - a molecule for all reasons. Q.J,* 2002, vol. 95, 3-13
- **DENHART DT ; GIACHELLI CM.** Rittling SR.2001. Role of osteopontin. *cellular signaling and toxicant injury,* vol. 41, 4723-49
- **SØRENSEN ES ; HØJRUP P ; PETERSEN T.** Post-translational modifications of bovine osteopontin: Identification of twenty-eight phosphorylation and three O-glycosylations sites. *Protein Sci.,* 1995, vol. 4, 2040-2049
- **SØRENSEN ES ; PETERSEN TE.** Identification of two phosphorylation motifs in bovine osteopontin. *Biochem.Biophys.Res.Comm.,* 1994, vol. 198, 200-205
- **SENGER DR ; PERRUZZI CA ; PAPADOPOULOS A ; TENEN DG.** *Biochem.Biophys.Acta.,* 1989, vol. 996, 43-48
- **DHANIREDDY R ; SENGER R ; MUKHERJEE BB ; MUKHERJEE AB.** *Acta Paediatr.,* 1993, vol. 82, 821-2

- **SORENSEN S ; JUSTESEN SJ ; JOHNSEN, AH.** *Protein Expression and Purification,* 2003, vol. 30, 238-245
- **CHOI, K. W. et al.** *Purification and properties of osteopontin from bovine milk*
- database. PREV200300375294
- **BAYLESS, K.J. et al.** Isolation and Biological Properties of Osteopontin from Bovine Milk. *Protein expression and purification,* April 1997, vol. 9 (3), 309-314
- **SORENSEN ES ; PETERSEN TE.** Purification and characterization of three proteins isolated from the proteose peptone fraction of bovine milk. *J.Dairy Res.,* 1993, vol. 60, 189-197
- **SENGER DR ; PERRUZZI CA ; PAPADOPOULOS A ; TENEN DG.** Purification of a milk protein closely similar to tumor-secreted phosphoproteins and osteopontin. *Biochem.Biophys.Acta,* 1989, vol. 996, 43-48